# EUROPÄISCHE PATENTANMELDUNG

(11) **EP 0 554 741 A1**
(43) Veröffentlichungstag der Anmeldung: **11.08.1993**
(21) Anmeldenummer: 93101056.5
(22) Anmeldetag: 25.01.1993
(51) Int. Cl.: C07D 209/48, C08G 73/16

(54) **Amidoimidobisphenole, deren Herstellung und daraus abgeleitete thermostabile Polymere**

(30) Priorität: 06.02.1992 AT 194/92
(71) Anmelder: DSM Chemie Linz GmbH, 4021 Linz (AT)
(72) Erfinder: Greber, Gerd, Dr., A-2540 Bad Vöslau (AT); Gruber, Heinrich, Dr., A-1190 Wien (AT); Sumper, Franz, Dipl.-Ing., A-2380 Perchtoldsdorf (AT)
(74) Vertreter: Kunz, Ekkehard, Dr.

(57) **Zusammenfassung**

Amidoimidobisphenole der Formel 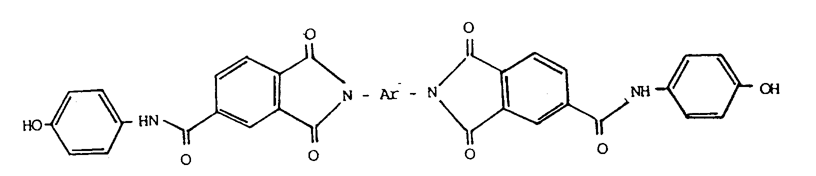
in der Ar ein unsubstituiertes, ein- oder mehrfach mit (C₁ - C₆)Alkyl, (C₁ - C₆)Alkoxy, Halogen oder Hydroxy substituiertes o, m oder p-Phenylen, Bisphenylen oder Naphthylen oder einen Rest der Formel II 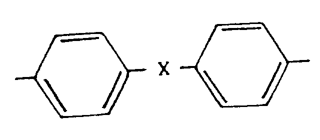
worin X ein gesättigter oder ungesättigter, linearer oder verzweigter (C₁ - C₆) Alkylenrest oder -O-, -S-, -SO-, -SO₂, - CO-, -COO-, -CO-NH-, -NH-, -(N-Alkyl)- mit 1 bis 20 Atomen, - (N-Aryl)- mit 6 - 60 C-Atomen oder -N=N- sein kann, bedeutet, deren Herstellung aus Trimellithsäureanhydrid und daraus durch Reaktion mit aliphatischen oder aromatischen Diestern oder Dichloriden gewonnene thermostabile Polyamidimidester.

## Beschreibung

Die Erfindung betrifft Amidoimidobisphenole, deren Herstellung und daraus abgeleitete thermostabile Polymere.

Die bis heute wichtigsten technisch angewandten thermostabilen Polymere stellen vollaromatische Polyimide dar. Diese Verbindungen weisen zwar die höchste Thermostabilität von allen Polymerwerkstoffen auf, besitzen jedoch aufgrund ihrer Unschmelzbarkeit und Unlöslichkeit Nachteile bezüglich ihrer Verarbeitbarkeit. Die etwas weniger thermostabilen Polyamide sind ebenfalls unschmelzbar und in den meisten organischen Lösungsmitteln unlöslich oder nur wenig löslich, so daß die Verarbeitung ebenfalls mit großen Schwierigkeiten verbunden ist.
Die schwierige Verarbeitbarkeit dieser Werkstoffe schränkt ihre möglichen Einsatzgebiete stark ein.
Durch den Einbau von Diaminen oder Dianhydriden mit flexiblen Gruppen, wie zum Beispiel -CH₂-, -O-, -S- oder -CO- oder aber von sterisch, anspruchsvollen Diaminen, beispielsweise in Polyamiden (US-PS 4,621,134), Polyimiden (JP-Kokai 1985-15228) oder Polyamidimiden (US-PS 4,724,257) wird zwar die Löslichkeit und/oder die Schmelzbarkeit und somit ihre Verarbeitbarkeit verbessert, die Thermostabilität der Polymere wird dadurch jedoch stark verringert.

Die eigentliche Problemlösung auf dem Gebiet der thermostabilen Polymeren liegt also noch immer auf der Entwicklung von Produkten mit hoher Thermostabilität und gleichzeitig guter Verarbeitbarkeit.

Aufgabe der vorliegenden Erfindung war es, neue Monomere zu entwickeln, die zu Polymeren führen, die bei hoher Thermostabilität löslich und/oder schmelzbar und somit nach herkömmlichen Methoden verarbeitbar sind.

Es wurde nun gefunden, daß diese Aufgabe mit Hilfe neuer Monomere auf der Basis von Aminophenol und Diimiddicarbonsäuren, die zu Polyamidimidestern mit hoher Thermostabilität, und guter Verarbeitbarkeit führen, gelöst werden kann.
Gegenstand der Erfindung sind demnach Amidoimidobisphenole der Formel I 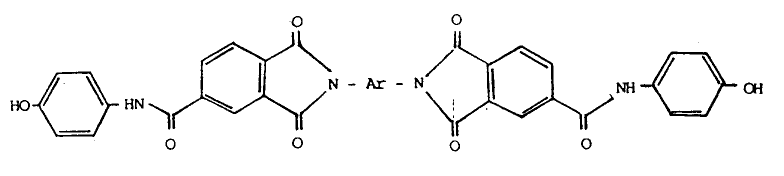
in der Ar ein unsubstituiertes, ein- oder mehrfach mit (C₁ - C₆)Alkyl, (C₁ - C₆)Alkoxy, Halogen oder Hydroxy substituiertes o, m oder p-Phenylen, Bisphenylen oder Naphthylen oder einen Rest der Formel II 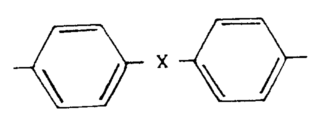
worin X ein gesättigter oder ungesättigter, linearer oder verzweigter (C₁ - C₆)Alkylenrest oder -O-, -S-, -SO-, -SO₂- - CO-, -COO-, -CO-NH-, -NH-, -(N-Alkyl)- mit 1 bis 20 C-Atomen, - (N-Aryl)- mit 6 bis 60 C-Atomen oder -N=N- sein kann,
bedeutet.
In der Formel I bedeutet Ar einen unsubstituierten, ein - oder mehrfach mit (C₁ - C₆)Alkyl, wie Methyl, Ethyl, Propyl, Butyl, Isobutyl oder Hexyl, (C₁ - C₆)Alkoxy, wie Methoxy, Ethoxy, Propoxy, Isopropoxy, Butoxy, oder mit Fluor , Chlor oder Brom oder mit Hydroxy substituierten o-, m-, p-Phenylen-, Bisphenylen- oder Naphtylenrest.

Ar kann auch einen Rest der Formel II, wie z. B. Diphenylenmethan, Diphenylenether, Diphenylensulfon, Diphenylensulfid oder Diphenylencarboxyd bedeuten.
Bevorzugt sind Verbindungen der Formel I, in der Ar einen unsubstituierten oder einfach mit Methyl, Ethyl, Methoxy oder Ethoxy, Chlor oder Hydroxy substituierten m- oder p-Phenylen- oder Bisphenylenrest bedeutet. In weiteren bevorzugten Verbindungen bedeutet Ar einen Rest der Formel II, worin X ein unsubstituiertes, lineares oder verzweigtes (C₁ - C₄)Alkylen, wie Methylen, Ethylen, Propylen, Butylen, Dimethylmethylen oder Dimethylethylen oder die Gruppe -O-, -S- oder -SO₂- sein kann.

Besonders bevorzugt sind Verbindungen der Formel I, in der Ar einen unsubtiuierten m- oder p-Phenylen- oder Bisphenylenrest oder einen Rest der Formel II, worin X die Gruppe -CH₂-, - C(CH₃)₂-, -SO₂-, -S-, oder -O- sein kann, bedeutet.

Ein weiterer Gegenstand der vorliegenden Erfindung ist ein Verfahren zur Herstellung von Verbindungen der Formel I in der Ar wie oben definiert ist, das dadurch gekennzeichnet ist,daß Diamine der Formel III

NH₂ - Ar -NH₂

in der Ar wie oben definiert ist, mit Trimellithsäureanhydrid der Formel IV 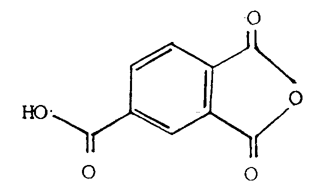
in einem unter Reaktionsbedingungen inerten Verdünnungsmittel zu Diimiddicarbonsäuren der Formel V 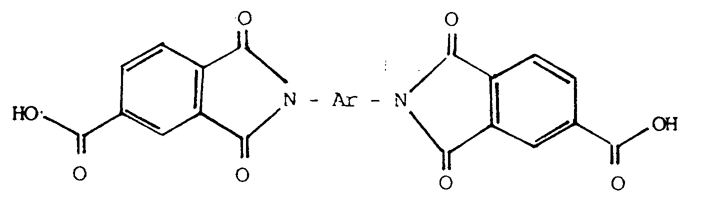
in der Ar wie oben definiert ist,
umgesetzt werden,
anschließend die Diimiddicarbonsäuren der Formel V in die entsprechenden Diimiddicarbonsäuredichloride der Formel VI 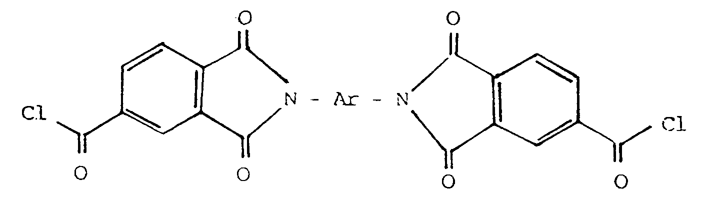
überführt werden, worauf durch Reaktion der Diimiddicarbonsäurechloride der Formel VI mit 4-Aminophenol, die Verbindungen der Formel I erhalten werden.

In der 1. Stufe werden Diamine der Formel III mit Trimellithsäureanhydrid umgesetzt. Diamine der Formel III sind beispielsweise o-, m- oder p-Phenylendiamin, 2,5- oder 2,4-Diaminotoluol, 2,4- oder 2,5-Diaminophenol, 4,4'-Diaminodiphenyldimethylmethan, 4,4'-Diaminodiphenylmethan, 4,4'-Diaminodiphenylsulfon, 4,4'-Diaminodiphenylether, 4,4'-Diaminodiphenylsulfid, 4,4'-Diaminobenzophenon, 4,4'-Diaminodiphenylsulfoxid, 4,4'-Diaminosulfon, p-Aminobenzoesäure-p-aminophenylester oder 4,4'-Diaminodiphenylamin.

Bevorzugt werden äquivalente Mengen der Reaktionspartner eingesetzt.

Die Umsetzung erfolgt vorzugsweise in einem unter Reaktionsbedingungen inerten Verdünnungsmittel, wie z.B. N-Methylpyrrolidon (NMP), Dimethylformamid (DMF), Dimethylacetamid (DMA), Dimethylsulfoxid (DMSO) oder Hexamethylphosphorsäuretriamid (HMP).
Bevorzugt wird DMF oder NMP als Verdünnungsmittel eingesetzt. Das Reaktionsgemisch wird dabei auf etwa 50° C - 70° C erwärmt und etwa 30 - 90 min lang auf dieser Temperatur gehalten. Die bei dieser Umsetzung intermediär entstehenden Amidsäuren werden durch 1- bis 4-stündiges, vorzugsweise 2- bis 3-stündiges Erhitzen auf Rückflußtemperatur cyclisiert, worauf man die so erhaltenen Diimiddicarbonsäuren der Formel V durch übliche Methoden, wie z.B. Kristallisation oder Ausfällen isoliert.
Gegebenenfalls können die isolierten Zwischenprodukte der Formel V durch bekannte Methoden, wie z.B. Umkristallisieren oder Umfällen gereinigt werden.

In dem zweiten Reaktionschritt erfolgt die Überführung der Dicarbonsäuren in ihre Dichloride nach bekannten Methoden, z.B. durch Reaktion mit Thionylchlorid, PCl₃ oder Sulfonylchlorid, in einem unter Reaktionsbedingungen inerten Verdünngungsmittel,wie z.B. NMP oder DMF bei Temperaturen von etwa -50 °C bis +10° C, vorzugsweise bei -25° C bis +5° C. Vorzugsweise werden äquivalente Mengen an Diimiddicarbonsäure und aktivem anorganischen Säurechlorid eingesetzt. Das so gebildete Diimiddicarbonsäuredichlorid muß nicht isoliert werden, sondern der Reaktionslösung kann sofort 4-Aminophenol, suspendiert in einem Verdünnungsmittel wie z.B. DMF oder NMP, zugegeben werden. Pro Mol Diimiddicarbonsäuredichlorid werden bevorzugt 2 Mol 4-Aminophenol eingesetzt, ein Überschuß an 4-Aminophenol stört jedoch nicht, überschüssiges 4-Aminophenol muß jedoch zurückgewonnen werden.
Zweckmäßigerweise wird dem Reaktionsgemisch eine geeignete Base wie z.B. Trimethylamin, Dimethylanilin oder Pyridin in äquivalenter Menge zugegeben, um die bei der Reaktion entstehende Salzsäure zu entfernen.
Anschließend wird das Reaktionsgemisch etwa 1 bis 3 Stunden bei -30° C bis +10° C, vorzugsweise bei -20° C bis O° C, gerührt und dann zur Vervollständigung der Reaktion bei Raumtemperatur weitere 1 bis 3 Stunden gerührt.
Die erfindungsgemäßen Amidoimidobisphenole können durch übliche Methoden, wie etwa Ausfällen, Kristallisation oder Zentrifugation, isoliert werden.

Die so isolierten Amidoimidobisphenole können, falls erforderlich, anschließend noch durch bekannte Methoden, wie z.B. Umkristallisieren, Umfällen oder Chromatographie, gereinigt werden.

Die erfindungemäßen Amidoimidobisphenole können dann zur Herstellung von thermostabilen Polymeren verwendet werden.

Ein weiterer Gegenstand der vorliegenden Erfindung sind demnach Polyamidimidester der Formel VII 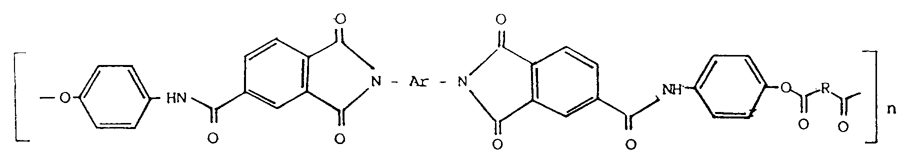
in der Ar, wie oben definiert ist
und R einen linearen oder verzweigten, gesättigten oder ungesättigten, unsubstituierten oder mit Amino, Chlor oder Hydroxy substituierten (C₁ - C₂₀) Alkylenrest oder ein unsubstituiertes oder mit Hydroxy, (C₁ - C₄)Alkyl, (C₁ - C₄)Alkoxy oder Amino substituiertes o-, m- oder p-Phenylen, Bisphenylen oder Naphthylen bedeutet.

Die Herstellung der Polymeren kann durch Grenzflächenpolykondensation oder Lösungspolykondensation in einem unter Reaktionsbedingungen inerten Verdünnungsmittel, wie etwa n-Hexan, Chloroform, Benzol, Toluol oder Chloroform-Wasser Gemisch, oder falls die Edukte schmelzbar sind, durch Polykondensation in der Schmelze, erfolgen.
Bevorzugt werden die Polymere durch Grenzflächenpolykondensation der Amidoimidobisphenole mit Dicarbonsäuredichloriden-, oder -diestern hergestellt.
Als Dicarbonsäuredichloride oder -diester werden beispielsweise Terephthalsäure-, Phthalsäure- oder Adipinsäurederivate eingesetzt.

Die Grenzflächenpolykondensation wird in Anwesenheit eines Phasentransferkatalysators, wie zum Beispiel N-Cetyl-N,N,N-trimethylammoniumbromid durchgeführt. Zweckmäßigerweise wird bei Verwendung der Dichloride dem Reaktionsgemisch eine geeignete Base, wie z.B. Natronlauge, zugegeben, um die bei der Reaktion entstehende Salzsäure zu entfernen.
Die Reaktionstemperatur liegt etwa zwischen O und 50° C, vorzugsweise bei etwa 5 - 30° C. Die Isolierung der Polymere kann durch übliche Methoden, wie z.B. Abfiltrieren oder Zentrifugieren erfolgen.

Die so hergestellten Polymere zeichnen sich durch hohe Thermostabilitäten und niedrige Glastemperaturen aus. Die gute Löslichkeit in NMP erleichtert die Verarbeitung zu Fasern oder Folien.

### Beispiel 1

### a) Herstellung von 1,4-Bis-(1,3-dihydro-1,3-dioxo-5-carboxy-2-(1H)isoindolyl)-benzol (S1)

10 g (54,04 mmol) Trimellithsäureanhydrid (TMA) und 2,81 (26,02 mmol) 1,4-Phenylendiamin wurden in 150 ml wasserfreiem NMP gelöst, erhitzt und eine Stunde lang auf 60 °C gehalten. Anschließend wurde auf die Rückflußtemperatur erhitzt und weitere 2 Stunden auf dieser Temperatur gehalten. Das Reaktionsgemisch wurde dann in 800 ml Aceton gegossen, der gebildete gelbe Niederschlag abgesaugt, mit Aceton gewaschen und getrocknet.

| | |
|---|---|
| Ausbeute: | 10,9 g (92 % d.Th.) |
| Schmelzpunkt: | 448 °C |

### b) Herstellung von 1,3-Bis-(1,3-dihydro-1,3-dioxo-5-carboxy-2-(1H)isoindolyl)-benzol (S2)

Die Herstellung erfolgte analog Beispie 1a)

| | |
|---|---|
| Ausbeute: | 10,3 g (87 % d.Th.) |
| Schmelzpunkt: | 395 °C |

### c) Herstellung von 4,4'-Bis-(1,3-dihydro-1,3-dioxo-5-carboxy-2-(1H)isoindolyl)-diphenylmethan (S3)

10 g (54,04 mmol) TMA und 5,16 g (26,02 mmol) 4,4'-Diaminodiphenylmethan wurden in 200 ml wasserfreiem DMF gelöst, für eine Stunde leicht erwärmt und weitere 2 Stunden auf Rückflußtemperatur gehalten. Zur Aufarbeitung wurde die Lösung mit Aceton versetzt, der gebildete Niederschlag abgesaugt und getrocknet. Das so erhaltene Rohprodukt wurde aus DMF umkristallisiert.

| | |
|---|---|
| Ausbeute: | 11,4 g (89 % d.Th.) |
| Schmelzpunkt: | 372 °C |

### d) Herstellung von 4,4'-Bis(1,3-dihydro-1,3-dioxo-5-carboxy-2-(1H)isoindolyl)-diphenylsulfon (S4) und 4,4'-Bis-(1,3-dihydro-1,3-dioxo-5-carboxy-2-(1H)isoindolyl)diphenylether

Die Herstellung erfolgte analog Beispiel 1c) **(S5)**

| | | |
|---|---|---|
| Ausbeute: | 10,1 g S4 (65 % d.Th.); | 12,9 g S5 (90 % d.Th.) |
| Schmelzpunkt: | 380 °C (S4); | 390 °C (S5) |

### Beispiel 2:

### Herstellung von 1,3-Bis-(1,3-Dihydro-1,3-dioxo-5-(4-hydroxyphenyl)-carboxamid-2-(1H)isoindolyl)-benzol (D2)

4 g (8,76 mmol) S2 wurden bei -20 °C in 100 ml wasserfreiem NMP suspendiert und 2,09 g (17,52 mmol) Thionylchlorid wurden durch ein Septum tropfenweise zugegeben. Nach etwa 1,5 Stunden hat sich das gebildete Säuredichlorid gelöst, worauf eine Lösung von 1,9 g (17,52 mmol) 4-Aminophenol und 1,4 g (17,52 mmol) wasserfreiem Pyridin in 50 ml wasserfreiem NMP innerhalb 30 Minuten zugeben wurde. Anschließend wurde das Reaktionsgemisch weitere 2 Stunden bei -25 bis -20 °C gerührt, dann auf Raumtemperatur erwärmt und weitere 2 Stunden nachreagieren gelassen.

Zur Isolierung des Produktes wurde die Reaktionslösung auf 1 l Wasser gegossen, der gebildete Niederschlag abzentrifugiert, getrocknet, mit Wasser gewaschen und zur Entfernung von NMP bei 150 °C im Trockenschrank aufbewahrt.

| | |
|---|---|
| Ausbeute: | 4,8 g D2 (86 % d.Th.) |
| Schmelzpunkt: | 333 °C |

### b) Herstellung von 4,4'-Bis-(1,3-Dinydro-1,3-dioxo-5-(4-hydroxyphenyl)-carboxamido-2-(1H)isoindolyl)-diphenylsulfon (D4) und 4,4'-Bis-(1,3-Dihydro-1,3-dioxo-5-(4-hydroxyphenyl)-carboxamido-2-(1H)isoindolyl)-diphenylehter (D5)

Die Herstellung erfolgte analog zu Beispiel 2a)

| | | |
|---|---|---|
| Ausbeute: | 4,3 g D4 (82 % d.Th.); | 4,6 g D5 (86 % d.Th.) |
| Schmelzpunkt: | 335 °C (D4); | 377 °C (D5) |

### c) Herstellung von 4,4'-Bis-(1,3-Dihydro-1,3-dioxo-5-(4-hydroxyphenyl)-carboxamido-2-(1H)isoindolyl)-diphenylmethan (D3)

Die Herstellung erfolgte analog Beispiel 2a, die Temperatur bei der Bildung des Säuredichlorides wurde jedoch auf 0 °C gehalten.

| | |
|---|---|
| Ausbeute: | 4,1 g (7,7 % d.Th.) |
| Schmelzpunkt: | 349 °C |

### Beispiel 3

### Herstellung von Polyamidimidester (TD2) aus D2 und Terephthalsäuredichlorid (TPDCl)

2 g (3,13 mmol)D2 wurden in einer Lösung von 0,5 g (12,5 mmol)NaOH gelöst und mit 1,10 g (3 mmol) des Phasentransferkatalysators (PTK) N-Cetyl,N,N,,N-trimethylammoniumbromid versetzt. Anschließend wurde eine Lösung von 0,64 g (3,13 mmol)TPDCl in 20 ml wasserfreiem Chloroform rasch zugegeben, wobei sich ein weißer Feststoff bildete.
Das ausgefallene Produkt wurde abfiltriert, mit Wasser gewaschen und getrocknet. Es wurden weitere Polyamidimidester (TD3, TD4, TD5) aus den Diolen D3, D4 und D5 und Polyamidimidester (ID2, ID3, ID4, ID5) aus den Diolen D2 - D5 und Isophthalsäuredichlorid (IPDCL) analog zu Beispiel 3 a) hergestellt. Aus den Diolen D2 - D4 und Adipinsäuredichlorid wurden ebenfalls Polyamidimidester (AD2, AD3, AD4) analog Beispiel 3a hergestellt. Als Lösungsmittel für das Dichlorid wurde jedoch anstelle von Chloroform n-Hexan verwendet.
Die Ausbeuten, Glastemperaturen, inhärenten Viskositäten, Thermostabilitäten, Löslichkeit und Folienbildungsvermögen aller Polyamidimidester sind in Tabelle 1 zusammengefaßt.

**Tabelle 1**

| **Polymer** | **Ausbeute [% d.Th.]** | **Tg [°C]** | **Tz [°C]** | **i.V. [dl/g]** | **L (NMP)** | **F** |
|---|---|---|---|---|---|---|
| TD2 | 87 | 220 | 415 | 0,51 | + | - |
| TD3 | 91 | 224 | 449 | 0,45 | + | + |
| TD4 | 85 | 259 | 429 | 0,32 | + | + |
| TD5 | 89 | 200 | 443 | 0,49 | + | + |
| ID2 | 99 | 204 | 375 | 1,20 | + | - |
| ID3 | 80 | 250 | 432 | 0,51 | + | + |
| ID4 | 84 | 237 | 413 | 0,44 | + | - |
| ID5 | 94 | 185 | 421 | 0,54 | + | + |
| AD2 | 79 | 215 | 330 | 0,22 | + | + |
| AD3 | 74 | - | 408 | 0,25 | + | + |
| AD4 | 87 | 236 | 389 | 0,23 | + | - |
| Tg: Glastemperatur Tz: Zersetzungstemperatur [Temperatur, bei welcher die Probe bei der Thermogravic Analysis 5% ihres Gewichtes verloren hat] i.V. inhärente Viskosität F: Folienbildungsvermögen | | | | | | |

## Patentansprüche

1. Verbindungen der Formel I 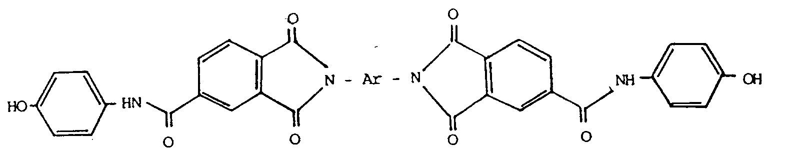 in der Ar ein unsubstituiertes, ein- oder mehrfach mit (C₁ - C₆)Alkyl, (C₁ - C₆)Alkoxy, Halogen oder Hydroxy substituiertes o-, m- oder p-Phenylen, Bisphenylen oder Naphthylen oder einen Rest der Formel II 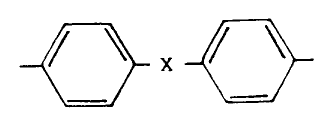 worin X ein gestättigter oder ungesättigter, linearer oder verzweigter (C₁ - C₆)Alkylenrest oder -O-, -S-, -SO-, -SO₂ -CO-, -COO-, -CO-NH-, -NH-, -(N-Alkyl)- mit 1 bis 20 Atomen, -(N-Aryl)- mit 6 bis 60 C-Atomen oder -N=N- sein kann,
bedeutet.

2. Verbindungen der Formel I, gemäß Anspruch 1, in der Ar einen unsubstituierten oder einfach mit Methyl, Ethyl, Methoxy, Ethoxy, Chlor oder Hydroxy substituierten m- oder p-Phenylen -oder Bisphenylenrest oder einen Rest der Formel II, worin X ein unsubstituiertes, lineares oder verzweigtes (C₁ - C₄)Alkylen oder eine Gruppe -O-, -S-, oder -SO₂ sein kann,
bedeutet.

3. Verbindungen der Formel I, gemäß Anspruch 1, in der Ar einen unsubstituierten m- oder p-Phenylen- oder Bisphenylenrest oder einen Rest der Formel II, worin X die Gruppe -CH₂-, - C(CH₃)₂-, -SO₂-, -S- oder -O- sein kann,
bedeutet.

4. Verfahren zur Herstellung von Verbindungen der Formel I, 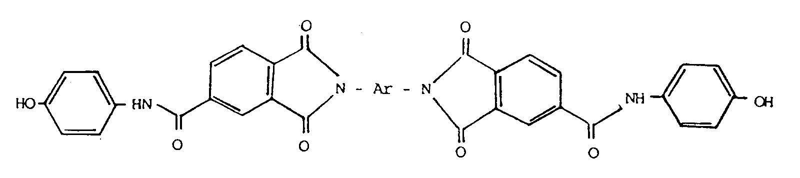 in der Ar wie im Anspruch 1 definiert ist,
dadurch gekennzeichnet, daß Diamine der Formel III
NH₂ - Ar - NH₂
in der Ar wie in Anspruch 1 definiert ist, mit Trimellithsäureanhydrid der Formel IV 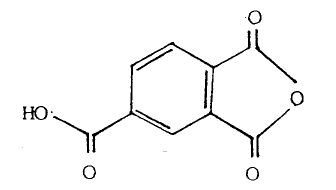 in einem unter Reaktionsbedingungen inerten Verdünnungsmittel unter Wasserabspaltung zu Diimiddicarbonsäuren der Formel V 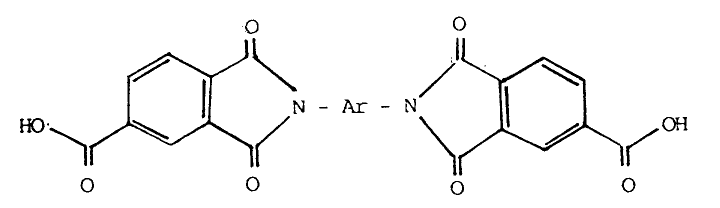 in der Ar wie in Formel III definiert ist, umgesetzt werden,
anschließend die gebildeten Diimiddicarbonsäuren der Formel V in die entsprechenden Diimiddicarbonsäuredichloride der Formel VI 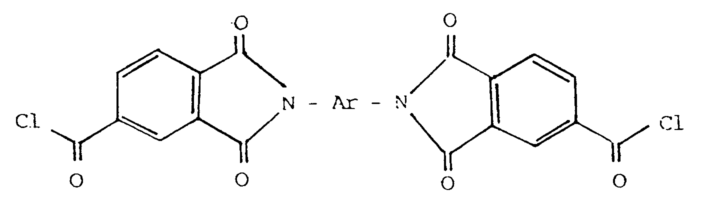 überführt werden,
worauf durch Reaktion der Diimiddicarbonsäuredichloride mit 4-Aminophenol die Verbindungen der Formel I erhalten werden.

5. Polyamidimidester der Formel VII 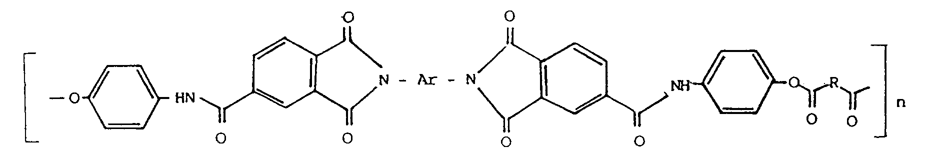 in der Ar wie in Anspruch 1 definiert ist, und R einen linearen oder verzweigten, gesättigten oder ungesättigten, unsubstituierten oder mit Amino, Hydroxy oder Chlor substituierten (C₁ - C₂₀)Alkylenrest oder ein unsubstituiertes oder mit Hydroxy, (C₁ - C₄)Alkyl, (C₁ - C₄)Alkoxy oder Amino, substituiertes o-, m- oder p-Phenylen, Bisphenylen oder Naphthylen bedeutet.

6. Polyamidimidester der Formel VII, gemäß Anspruch 5, in der Ar wie in Anspruch 3 definiert ist
und R einen linearen unsubstituierten (C₁ - C₈)-Alkylenrest oder ein unsubstituiertes m- oder p-Phenylen bedeutet.

7. Verfahren zur Herstellung von Verbindungen der Formel VII, 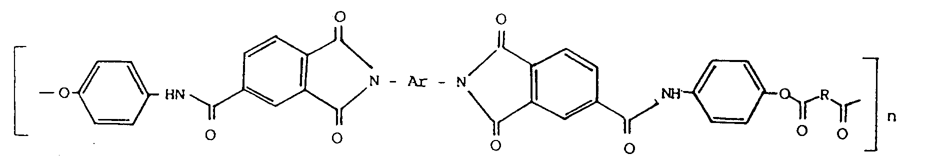 in der Ar wie im Anspruch 5 definiert sind,
dadurch gekennzeichnet, daß Verbindungen der Formel I,
in einem unter Reaktionsbedingungen inertem Verdünnungsmittel mit Verbindungen der Formel VIII
V-R-Z
worin R wie in Anspruch 5 definiert ist, und V und Z unabhängig voneinander COCl, oder COOR₁, wobei R₁ (C₁ - C₄) Alkyl sein kann, umgesetzt werden.
